# EUROPEAN PATENT SPECIFICATION

(11) **EP 0 808 299 B1**
(45) Date of publication and mention of the grant of the patent: **28.03.2001**
(21) Application number: 96907037.4
(22) Date of filing: 09.02.1996
(51) Int. Cl.: C07C 2/64, C07C 5/52, C07C 15/067, C07C 15/08

(54) **HEAVY AROMATICS PROCESSING**
BEHANDLUNG SCHWERER AROMATEN
TRAITEMENT D'AROMATIQUES LOURDS

(30) Priority: 10.02.1995 US 386892
(43) Date of publication of application: 26.11.1997
(73) Proprietor: MOBIL OIL CORPORATION, Fairfax, Virginia 22037-0001 (US)
(72) Inventor: BUCHANAN, John, Scott, Trenton, NJ 08619 (US); CHESTER, Arthur, Warren, Cherry Hill, NJ 08003-3009 (US); FUNG, Shiu, Lun, Anthony, Wilmington, DE 19810-3620 (US); KINN, Timothy, Frederick, Humble, Texas 77346 (US); MIZRAHI, Sadi, Cherry Hill, NJ 08034 (US)
(74) Representative: Kador & Partner
(86) International application number: PCT/US96/01818
(87) International publication number: WO 96/24568

(56) References cited:
- US-A- 4 016 218
- US-A- 4 127 471
- US-A- 4 418 235
- US-A- 5 406 016

## Description

This invention relates to a process for converting a feedstock containing heavy aromatics, specifically, C₉+ aromatics, to lighter aromatic products, specifically xylenes.

Para-xylene is an important by-product of petroleum refining because it is used in significant quantities for the manufacture of terephthalic acid which is reacted with polyols such as ethylene glycol in the manufacture of polyesters.

The major source of para-xylene is catalytic reformate which is prepared by mixing petroleum naphtha with hydrogen and contacting the mixture with a strong hydrogenation/dehydrogenation catalyst such as platinum on a moderately acidic support such as a halogen treated alumina.

Usually, a C₆ to C₈ fraction is separated from the reformate, extracted with a solvent selective for aromatics or aliphatics to separate these two kinds of compounds and produce a mixture of aromatic compounds which is relatively free of aliphatics. This mixture of aromatic compounds usually contains benzene, toluene and xylenes (BTX) along with ethyl benzene.

Liquids from extremely severe thermal cracking, e.g. high temperature steam cracking of naphtha are also rich in aromatics and may be used to prepare BTX in a similar manner.

Concentrated aromatic fractions are also provided by severe cracking over such catalysts as ZSM-5 and by conversion of methanol over ZSM-5.

Refineries have focused on the production of xylenes by transalkylation of C₉+ aromatics, which would normally only be of value as fuel, and toluene, over zeolite containing catalysts. The stability and transalkylation selectivity of zeolite beta for this reaction is the subject of several recent publications. See Das et al. "Transalkylation and Disproportionation of Toluene and C₉ Aromatics over Zeolite Beta" 23 Catalyst Letters pp. 161-168 (1994); Das et al. "Zeolite Beta Catalyzed C₇ and C₉ Aromatics Transformation" 116 Applied Catalysis A: General, pp. 71-79 (1994) and Wang et al. "Disproportionation of Toluene and of Trimethylbenzene and Their Transalkylation over Zeolite Beta", 29 Ind. Eng. Chem. Res. pages 2005-2012 (1990).

Additionally, processes for producing xylenes from hydrocarbon fractions containing substituted aromatics have been disclosed in the patent literature. U.S. Patent No. 4,380,685 discloses the para-selective alkylation, transalkylation or disproportionation of a substituted aromatic compound to provide a mixture of dialkylbenzene compounds employing as a catalyst a zeolite characterized by a Constraint Index of 1 to 12 and a silica/alumina mole ratio of at least 12/1, the catalyst having incorporated thereon various metals and phosphorus.

During the dealkylation reactions that, typically, accompany the conversion of heavy aromatics to xylenes, olefins are formed which tend to undergo secondary reactions resulting in the formation of coke which rapidly deactivates the catalyst and of undesirable aromatic byproducts which can also contribute to catalyst deactivation. One approach for solving the problem posed by olefins formation has been to encourage olefin saturation. Hydrogenation metals, such as platinum, are known to saturate olefins and prevent coke formation and have been incorporated into the catalysts. Similarly, employing high hydrogen partial pressures or high hydrogen to hydrocarbon mole ratios have been proposed to minimize olefin formation and catalyst aging.

U.S. Patent No. 5,030,787 discloses a process for the vapor-phase conversion of a feedstock containing at least one C₉+ aromatic compound to a product containing substantial quantities of C₆ to C₈ compounds, e.g. benzene and xylenes. The conversion occurs over a catalyst which contains a zeolite having a Constraint Index of 1 to 3, e.g. zeolites MCM-22, ZSM-12 and zeolite beta. Steam treatment of the zeolite is proposed, see Col. 9, lines 66-67. A Group VIII metal can be included with the catalyst. In the specific examples of the disclosure the zeolite is subjected to the steam treatment prior to incorporation of the hydrogenation metal, see Examples 20-22.

US-A-4,418,235 discloses transalkylation of aromatic hydrocarbons with a catalyst such as ZSM-12 that can contain a hydrogenation component. US-A-4,127,471 discloses treatment of catalysts with sulfur.

However, the use of hydrogenation components and high hydrogen partial pressures not only reduces olefin formation and catalyst aging but also promotes saturation of the aromatic compounds resulting in low yields of the desirable lighter aromatics products such as benzene, toluene and xylenes. Also, maintaining a high hydrogen to hydrocarbon mole ratio requires large reactors which are costly to manufacture and maintain. An object of the present invention is to obviate or alleviate these disadvantages.

The invention is directed to a process for converting C₉+ aromatic hydrocarbons to lighter aromatic products comprising the step of contacting a feed comprising the C₉+ aromatic hydrocarbons, benzene and/or toluene with a catalyst composition comprising a zeolite beta, ZSM-12 or MCM-22 and and a hydrogenation component to produce a product comprising xylenes, wherein the catalyst composition having the hydrogenation component is treated with a source of sulfur or with steam to reduce its aromatic hydrogenation activity.

ZSM-12 is more particularly described in U.S. Patent No. 3,832,449 and has a Constraint Index of 2.3 (316°C). The method in which Constraint Index is determined is described in US-A-4,016,218. Zeolite Beta is more particularly described in U.S. Patent No. Re. 28,341 (of original U.S. patent No. 3,308,069) and has a Constraint Index of 0.6-2.0 (316-399°C). Zeolite MCM-22 is described in U.S. Patent No. 4,954,325 and has a Constraint Index of 1.5 (454°C).

It may be desirable to incorporate the selected zeolite catalyst with another material which is resistant to the temperatures and other conditions employed in the process of this invention. Such materials include active and inactive materials and synthetic or naturally occurring zeolites as well as inorganic materials such as clays, silica and/or metal oxides such as alumina. The latter may be either naturally occurring or in the form of gelatinous precipitates or gels including mixtures of silica and metal oxides. Use of a material in conjunction with the zeolite catalyst, i.e. combined therewith or present during its synthesis, which itself is catalytically active, may change the conversion and/or selectivity of the catalyst.
Inactive materials suitably serve as diluents to control the amount of conversion so that transalkylated products can be obtained economically and orderly without employing other means for controlling the rate of reaction. These materials may be incorporated into naturally occurring clays, e.g. bentonite and kaolin to improve the crush strength of the catalyst under commercial alkylation operating conditions. The materials, i.e. clays, oxides, etc. function as binders for the catalyst. It is desirable to provide a catalyst having good crush strength because in commercial use, it is desirable to prevent the catalyst from breaking down into powder-like materials. These clay binders have been employed normally only for the purpose of improving the crush strength of the catalyst.

Naturally occurring clays which can be composited with the zeolite catalyst herein include the montmorillonite and kaolin family, which families include the subbentonites, and the kaolins commonly known as Dixie, McNamee, Georgia and Florida clays or others in which the main mineral constituent is halloysite, kaolinite, dickite, nacrite or anauxite. Such clays can be used in the raw state as originally mined or initially subjected to calcination, acid treatment or chemical modification. Binders useful for compositing with zeolite also include inorganic oxides, notably alumina.

In addition to the foregoing materials, the zeolite catalyst can be composited with a porous matrix material such as an inorganic oxide selected from the group consisting of silica, alumina, zirconia, titania, thoria, beryllia, magnesia, and combinations thereof such as silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia. It may also be advantageous to provide at least a part of the foregoing matrix materials in colloidal form so as to facilitate extrusion of the bound catalyst component(s).

The relative proportions of finely divided crystalline material and inorganic oxide matrix vary widely, with the crystal content ranging from 1 to 95 percent by weight and more usually, particularly when the composite is prepared in the form of beads, in the range of 2 to 80 weight percent of the composite. The zeolite is employed in combination with a hydrogenation component such as a metal selected from Group VIII of the Periodic Table of the Elements (CAS version, 1979). Specific examples of useful hydrogenation materials are iron, ruthenium, osmium, nickel, cobalt, rhodium, iridium, or a noble metal such as platinum.

The amount of the hydrogenation component is selected according to a balance between hydrogenation activity and catalytic functionality. Less of the hydrogenation component is required when the most active metals such as platinum are used as compared to molybdenum which does not possess such strong hydrogenation activity. Generally, the catalyst contains 0.01 to 10 wt.%, preferably 0.05 to 5 wt%, of the hydogenation component.

The hydrogenation component can be incorporated into the catalyst composition by co-crystallization, exchanged into the composition to the extent a Group IIIA element, e.g., aluminum, is in the structure, impregnated therein or mixed with the zeolite and the inorganic oxide matrix. Such component can be impregnated in, or on, the zeolite such as for example, in the case of platinum, by treating the zeolite with a solution containing a platinum metal-containing ion. Suitable platinum compounds for impregnating the catalyst with platinum include chloroplatinic acid, platinous chloride and various compounds containing platinum amine complex, such as Pt(NH₃)₄Cl₂.H₂O.

After treatment with the hydrogenation function, the catalyst composite is usually dried by heating the catalyst at a temperature of 150 to 320°F (65 to 160°C), preferably from 230°F to 290°F (110°C to 143°C) for at least about 1 minute and generally not longer than about 24 hours. Thereafter, the catalyst composite is calcined in a stream of dry gas, such as air or nitrogen at temperatures of 500 °F to 1200°F (260°C to 649°C) for 1 to 20 hours. Calcination is preferably conducted at pressures ranging from 15 to 30 psia (100-200 kPa).

The catalyst composition is treated to reduce its aromatics hydrogenation activity, without substantially inhibiting its olefin saturation activity which prevents formation of the desirable products.

Aromatics loss over the treated catalyst composition of this invention is substantially lower than the aromatics loss sustained over the untreated catalyst.

The activity of the catalyst composition for aromatics ring loss relative to the entire amount of aromatics in the feed, is an effective way to evaluate the aromatics hydrogenation activity of the catalyst. Ideally, aromatics ring loss is less than 1 mole %. However, ring losses of less than 10 mole %, specifically, less than 5 mole %, even more specifically, less than 2 mole %, are acceptable, based on the entire amount of aromatics in the feed. Ring loss is determined using gas chromatography by comparing the amount of aromatics in the feed with the amount of aromatics in the product.

The benzene hydrogenation activity test (BHA test) can also be used to determine catalytic activity for hydrogenation of benzene to cyclohexane and it is a good indicator of aromatics hydrogenation capability which, for purposes of the instant invention is minimized. The test is, typically, used to determine the activity of noble metal catalysts. The conditions of the BHA test are described below in the examples. The BHA test is also described in U.S. Patent Nos. 5,188,996; 4,952,543; 4,837,397 and 4,849,385. This test provides a rate of benzene hydrogenation in terms of moles benzene/moles hydrogenation functionality/hour at 100°C. After treatment the catalyst of the invention has a BHA less than 500, and preferably less than 400, most preferably less than 20. The extent and methods of treatment of the catalyst including the hydrogenation functionality for minimizing loss of aromatics may vary depending upon the catalyst composition and its method of manufacture, e.g. the method of incorporating the hydrogenation functionality.

Steam treatment of the catalyst composition is an effective method for minimizing the aromatics hydrogenation activity of the catalyst composition. In the steaming process the catalyst is, usually, contacted with 5 to 100% steam at a temperature of 500°F to 1200°F (260 to 910°C) for 1 to 20 hours at a pressure of 100 to 2500 kPa.

Effective treatment with sulfur is accomplished by contacting the catalyst with a source of sulfur at a temperature of 600 to 900°F (316 to 480°C). The source of sulfur can be contacted with the catalyst via a carrier gas, typically, an inert gas such as hydrogen or nitrogen. In this embodiment, the source of sulfur is typically hydrogen sulfide.

Catalyst treatment can be effected ex-situ prior to the process of the invention or can be effected in situ in the process reactor either before or during at least part of the process.

For example, a source of sulfur can be co-fed with the hydrocarbon feedstream in a concentration ranging from 50 ppmw sulfur to 10,000 ppmw sulfur. Any sulfur compound that will decompose to form H₂S and a light hydrocarbon at about 900°F (480°C) or less will suffice. Typical examples of appropriate sources of sulfur include carbon disulfide and alkylsulfides such as methylsulfide, dimethylsulfide, dimethyldisulfide, diethylsulfide and dibutyl sulfide. Sulfur treatment can be considered sufficient when sulfur breakthrough occurs; that is, when sulfur appears in the liquid product.

Typically, sulfur treatment is initiated by incorporating a source of sulfur into the feed and continuing sulfur treatment for a few days, typically, up to 10 days, more specifically, from one to five days. The sulfur treatment can be monitored by measuring the concentration of sulfur in the product off gas. During this treatment, the sulfur concentration in the off gas should range from 20 to 500 ppmw sulfur, preferably 30 to 250 ppmw.

A further method for minimizing the aromatics hydrogenation activity of the catalyst composition is by the addition of an inactive or less active element, such as an element selected from Group IB of the Periodic Table of the Elements (CAS Version, 1979). The IB element specifically contemplated is copper.

Any one or a combination of these in situ and/or ex situ methods can be employed for minimizing the aromatics hydrogenation activity of the catalyst. It has been found that these methods minimize aromatics hydrogenation activity while sustaining sufficient hydrogenation of olefins which avoids rapid catalyst aging.

Aromatics hydrogenation activity is also controlled by operating the process under conditions of low hydrogen partial pressure. Typically, an appropriately low hydrogen partial pressure is 100 to 3000 kPa, preferably 700 to 2100 kPa and hydrogen to hydrocarbon mole ratio of less than 3.0, preferably 1.0 to 2.0.

The heavy aromatics feed used in this process comprises one or more aromatic compounds containing at least 9 carbon atoms such as, e.g. trimethylbenzenes, dimethylbenzenes, and diethylbenzenes, etc. Specific C₉+ aromatic compounds include mesitylene (1,3,5-trimethylbenzene), durene (1,2,4,5-tetramethylbenzene), hemimellitene (1,2,4-trimethylbenzene), pseudocumene (1,2,4-trimethylbenzene), 1,2-methylethylbenzene, 1,3-methylethylbenzene, 1,4-methylethylbenzene, propyl-substituted benzenes, butyl-substituted benzenes, isomers of dimethyl-ethylbenzenes, etc.

Suitable feeds include a C₉+ refinery fractions which are rich in aromatics, that is contain at least 80 wt.% C₉+ aromatics. Typical refinery fractions which may be useful include catalytic reformate, FCC naphtha or TCC naphtha.

The feedstock employed contains benzene and/or toluene in addition to the C₉+ compounds. The feed can also contain xylenes. This charge will normally constitute 40% to 90 %, more specifically 50 to 70 %, by volume of the entire feed, the balance of the feed is made up by C₉+ aromatics.

The process can be conducted in any appropriate reactor including a radial flow, fixed bed, continuous down flow or fluid bed reactor.

The process is performed at a temperature of 600°F to 1100°F (315°C to 590°C), preferably 700°F to 950°F (370°C to 510°C) a catalyst inventory of 0.5 to 4.0 WHSV and a total system pressure of 50 to 1000 psig (450 to 7000 kPa).

The invention will now be more particularly described with reference to the accompanying drawings, in which:

Figure 1 is a simplified schematic flow diagram of one embodiment of the process of this invention.

Figure 2 is a plot of hydrocarbon conversion at constant pressure and hydrogen to hydrocarbon mole ratio at a temperature of about 725°F (385°C) and a temperature of about 850°F (454°C) as a function of days on stream vs. average reactor temperature.

Figure 3 is a plot of hydrocarbon conversions at constant temperature and pressure at hydrogen to hydrocarbon mole ratios of 2:1 and 3:1 as a function of days on stream vs. average reactor temperature.

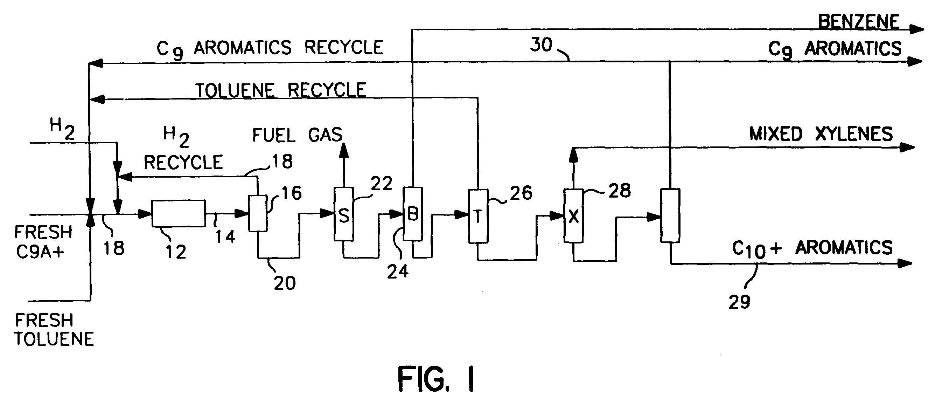

Referring to Figure 1, a C₉+ aromatics stream along with toluene and hydrogen are introduced via line 10 to reactor 12 which contains the transalkylation catalyst of this invention. The reactor is maintained under conditions sufficient so that benzene and methyl aromatics (toluene, xylenes, trimethylbenzenes and tetramethylbenzenes) approach thermodynamic equilibrium through transalkylation reactions. The C₈ to C₁₁ aromatics having C₂+ alkyl groups undergo dealkylation to form light gas and benzene, ethylbenzene and methylbenzenes which can undergo transalkylation reactions. The conditions are maintained to promote the secondary reactions which involve hydrogenation of light olefins which reduce coke. The conditions are also maintained to prevent olefins from taking part in formation of heavy aromatics compounds that can deactivate the catalyst or produce undesirable byproducts. These results are achieved without producing a high yield of saturated aromatics. The product of reactor 12 is withdrawn via line 14 and introduced to hydrogen separator 16 which separates hydrogen for recycle to reactor 12 via line 18. The feed then passes via line 20 to a stabilizer section 22 which removes C₅- fuel gas by known techniques. Thereafter, the product is fractionated into benzene, toluene and xylenes streams in fractionators 24, 26 and 28, respectively, for separation of these streams. The remaining product which comprises unreacted C₉+ feed and any heavy aromatics is separated into a C₉ aromatics stream 30 and a C₁₀+ aromatics stream 29. Stream 30 is recycled back to the reactor feed, removed from the process, or a combination of both (partial recycle). The C₁₀+ aromatics stream 29 is suitable for gasoline blending or other product such as solvents.

### Example 1

This example demonstrates formation of a platinum exchanged alumina bound ZSM-12 catalyst.

65 parts of ZSM-12 synthesized according to U.S. Patent No. 3,832,449 was mixed with 35 parts of LaRoche Versal 250 alumina on a dry basis. The mixture was dry mulled and formed into 1/16" (1.6mm) cylindrical extrudates. The extrudates were dried, activated and calcined. Platinum (0.1 wt.%) was exchanged into the extrudates using [(NH₃)₄Pt]Cl₂. The extrudates were washed, dried and calcined at 660°F (350°C). The platinum containing calcined extrudates were steamed at 900°F (480°C) for 4 hours. The resulting catalyst was designated as catalyst A and had an alpha activity of 53 and a surface area of 281 m²/g.

The catalyst was tested for its activity for hydrogenation of benzene to cyclohexane in the BHA test. In this test, a gaseous mixture containing 100:1 molar ratio of hydrogen and benzene was flowed through a vertical quartz ("Vycor" trademark) tubular reactor, 1/4 inch (6 mm) in diameter and 5 inches (12 cm) in length, containing about 250 mg of the catalyst, at a hydrogen flow rate of 200 cc/min, a total pressure of 1 atm. and temperatures between 75°F (24°C) and 300°F (150°C), depending on the activity of the catalyst. The benzene hydrogenation activity was determined by measuring the ability of the catalyst to hydrogenate benzene in terms of the moles of benzene hydrogenated per moles of platinum per hour at 100°C. The catalyst had a BHA of 444 moles benzene/moles Pt/hour.

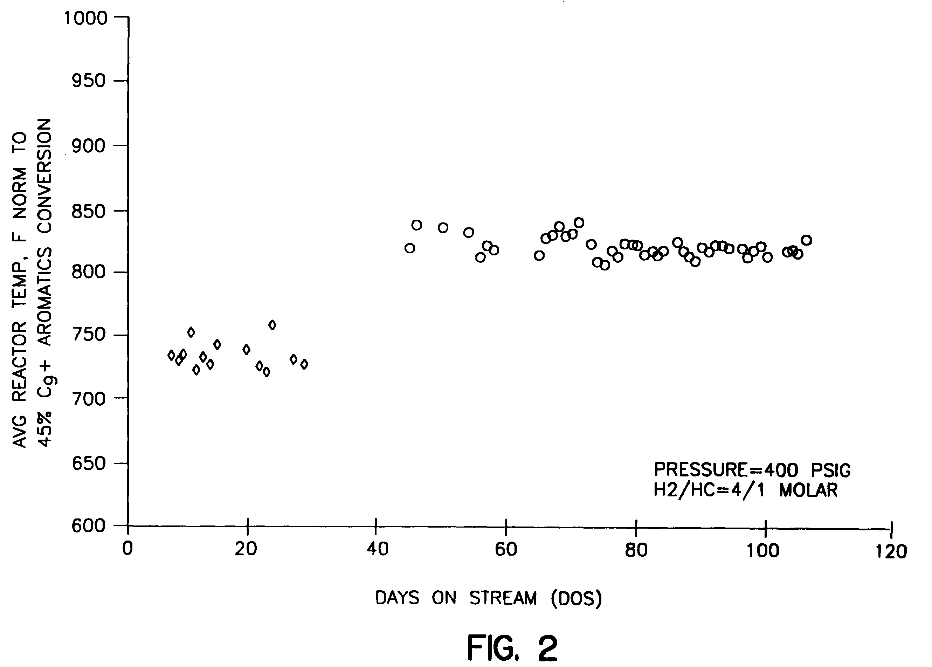

Figure 2 is a plot of average reactor temperature vs. days on stream which compared the performance of sulfided catalyst A at a temperature of about 725°F (385°C) and a temperature of about 850°F (454°C) at constant pressure of 400 psig (2860 kPa) and hydrogen to hydrocarbon mole ratio of 4:1. The plot shows that the catalyst remained stable at both temperatures within a period from about 10 to 100 days. Elevating the temperature to 850°F (454°C) after 40 days achieved a greater C₉+ conversion which remained relatively constant for up to 55 days longer.

### Example 2

This example demonstrates formation of a platinum impregnated alumina bound ZSM-12 catalyst.

65 parts of ZSM-12 (dry basis) synthesized according to U.S. Patent No. 3,832,449 were mixed in a muller with 35 parts of LaRoche Versal 250 alumina (dry basis) and with a platinum-containing solution using [(NH₃)₄Pt)Cl₂. The amount of platinum used gave a nominal loading of 0.1 wt.% (dry basis). The mixture was mulled and formed into 1/16" (1.6mm) cyclindrical extrudates. The extrudates were dried, activated and calcined. The platinum containing calcined extrudates were steamed at 900°F (480°C) for 4 hours. The platinum loading of the finished catalyst was 0.09 wt.%. The resulting catalyst was designated as catalyst B. The finished catalyst had an alpha activity of 77, a BHA of 118 moles benzene/moles of Pt/hour, and a surface area of 280 m²/g.

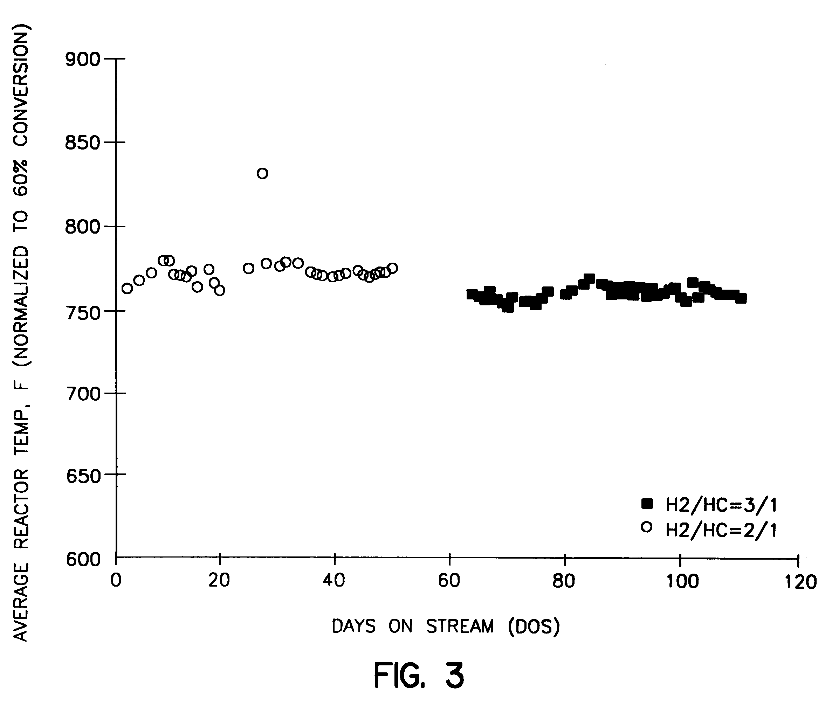

Figure 3 is a plot of average reactor temperature vs. days on stream which compares the performance of catalyst B at a low hydrogen to hydrocarbon mole ratio (2:1) with the performance of the same catalyst at a higher hydrogen to hydrocarbon mole ratio (3:1). Catalyst B was steamed and sulfided by exposing the catalyst to H₂S in a concentration ranging from between 0.05 and 4.0 wt.% in flowing hydrogen, as carrier, at temperatures ranging from about 650°F (340°C) and 800°F (430°C) until H₂S was detected in the product gas at a level of approximately 250 ppmw. The plot shows that catalyst performance is relatively stable over a period of over 100 days on stream at a relatively constant temperature of 750 to 775°F (400 to 413°C). This plot demonstrates how a low hydrogen to hydrocarbon mole ratio during the start-up phase of the conversion enhances catalyst stability.

### Examples 3-4

These examples compare the performance of catalyst A which was steamed after addition of the metal (Example 3) with a catalyst made in accordance with Example 22 of U.S. Patent No. 5,030,787 which was steamed prior to incorporation of the metal (comparative Example 4) in transalkylating C₉+ aromatics and toluene. The results are reported in Table 1.

**Table 1**

| Conversion of C₉+ Aromatics and Toluene Over Steamed Catalysts | | | | |
|---|---|---|---|---|
| | Example 3 | | Comparative Example 4 | |
| **Reaction conditions** | | | | |
| Hydrogenation metal | Steamed | | Unsteamed | |
| Pressure (psig) | 300 | | 250 | |
| (kPa) | (2170) | | (1825) | |
| | | | | |
| Temperature (°F) | 800 | | 800 | |
| (°C) | (430) | | (430) | |
| | | | | |
| WHSV (hr⁻¹) | 2.5 | | 2.6 | |
| | | | | |
| H₂:Hydrocarbon mole ratio | 1/1 | | 1/1 | |

| **Composition (wt.%)** | **Feed** | **Product** | **Feed** | **Product** |
|---|---|---|---|---|
| C₆ - C₈ Aromatics | 48.5 | 70.1 | 62.7 | 77.4 |
| C₉ aromatics | 43.0 | 20.5 | 30.4 | 11.0 |
| C₁₀ aromatics | 7.8 | 4.3 | 4.8 | 3.1 |
| Aromatic Ring | -- | 98.9 | -- | 96.7 |
| Retention, mol. % (loss) | | (1.1) | | (3.3) |

The data reported in Table 1 show that ring retention is significantly better when the hydrogenation metal is steamed as in Example 3. Additionally, the steamed hydrogenation functionality of Example 3 enabled a lower C₆ to C₈ aromatics feed to be converted to a product containing an amount of C₆ to C₈ aromatics which was comparable to the amount of C₆ to C₈ aromatics produced over the higher aromatics feed of Example 4. Moreover, although not reported in Table 1, of the C₆ to C₈ aromatics produced in Example 3, 36.0 wt.% were xylenes. In contrast, of the C₆ to C₈ aromatics produced in Example 4, only 28.6 wt.% were xylenes.

### Examples 5-6

These examples demonstrate the advantage of sulfiding catalyst A prior to introduction of the feed and also compare the performance of the presulfided catalyst with a presulfided catalyst which is further treated by adding sulfur to the feed. In both examples catalyst A was employed and the conditions of reaction included a temperature of 800°F (430°C), pressure of 400 psig (2860 kPa), WHSV of 2.5 and hydrogen to hydrocarbon mole ratio of 4. In Example 5, sulfiding was accomplished by contacting the steamed platinum exchanged ZSM-12 catalyst with about 50 cc/minute of 2% H₂S in hydrogen gas for about 40 minutes at 660°F to 750°F (350°C to 400°C). In Example 6, the catalyst was further sulfided in situ by cofeeding 600 ppm sulfur (in the form of dibutyl sulfide) with the hydrocarbon feed for two hours. The results of conversion over these sulfided catalysts are shown below in Table 2.

**Table 2**

| Conversion of C₉+ Aromatics and Toluene Over Steamed and Sulfur Treated Catalyst A | | | |
|---|---|---|---|
| **Product, wt.% of Feed** | **Feed** | **Example 5** | **Example 6** |
| C₅- | 0 | 36.7 | 26.2 |
| C₆+, non-aromatics | 0.4 | 1.3 | 1.0 |
| Benzene | 0 | 4.4 | 6.4 |
| Toluene | 56.0 | 19.1 | 24.0 |
| Ethylbenzenes | 0.1 | 0.8 | 1.0 |
| Xylenes | 1.8 | 25.2 | 28.0 |
| C₉+ Aromatics | 41.7 | 16.2 | 16.0 |
| **Aromatic Ring Retention, wt.%** **(loss)** | | 66.0 (44.0) | 75.0 (25.0) |

As the data in Table 2 show there are significant advantages, particularly in the aromatic ring retention and xylenes production, to cofeeding sulfur.

### Examples 7 and 8

These examples demonstrate that a lower aromatics saturation activity of the hydrogenation functionality can be established by operating the process at a low hydrogen to hydrocarbon mole ratio. In these examples, Catalyst A was employed in the transalkylation of a C₉+ aromatic hydrocarbon feedstream and toluene. The catalyst was not exposed to sulfur treatment.

**Table 3**

| Conversion of C₉+ Aromatics and Toluene Under Different Hydrogen:Hydrocarbon Mole Ratios | | | |
|---|---|---|---|
| | | Example 7 | Example 8 |
| **Reaction Conditions** | | | |
| Pressure, psig (kPa) | | 400 (2860) | 400 (2860) |
| Ave. Temperature, °F (°C) | | 780 (415) | 811 (433) |
| Time On Stream, days | | 3.1 | 6 |
| Space Velocity, WHSV | | 2.4 | 2.4 |
| H₂ to hydrocarbon mole ratio | | 2:1 | 1:1 |

| **Product, Wt.% of Feed** | **Feed** | **Product** | **Product** |
|---|---|---|---|
| C₅- | -- | 13.9 | 4.0 |
| C₆+ non-aromatic | 0.1 | 1.0 | 0.1 |
| Benzene | -- | 7.7 | 10.8 |
| Toluene | 57.0 | 29.0 | 34.8 |
| Ethylbenze ne | 0.1 | 1.8 | 1.5 |
| Xylenes | 1.7 | 26.0 | 28.6 |
| C₉+ Aromatics | 41.1 | 22.0 | 20.4 |
| **Aromatic Ring Retention, mol %** **(loss)** | | 87.1 (12.9) | 98.7 (1.3) |

### Examples 9-11

These examples demonstrate continuously cofeeding sulfur to treat the catalyst in situ.

In these examples a Pt/ZSM-12 catalyst was presulfided with about 50 cc/min 2% H₂S/H₂ for about 40 minutes at 660-750°F (350-400°C). In each example the reaction was operated at a temperature of 800°F (430°C), pressure of 300 psig (2170 kPa), WHSV of 2.5 and hydrogen to hydrocarbon mole ratio of 2. After about 125 hours on stream 100 ppmw sulfur was added to the feed and the sulfur was continuously cofed through about 170 hours on stream, at which time the sulfur feed was discontinued to evaluate the effect that continuous addition of sulfur had on the product. Product samples were analyzed at 134 hours on stream (Example 9) at 158 hours on stream (Example 10) and at 206 hours on stream (Example 11). The following Table 4 reports the results of product analysis.

**Table 4**

| Continuous Sulfur Addition | | | | |
|---|---|---|---|---|
| Example | | 9 | 10 | 11 |
| Sulfur Cofeed | | Yes | Yes | No |
| Time on Stream, hours | | 134 | 158 | 206 |
| Product, wt.% of Feed | | | | |

| | Feed | Product | Product | Product |
|---|---|---|---|---|
| C₅- | 0 | 6.1 | 6.0 | 7.3 |
| C₆+ Non aromatics | 0 | 0 | 0 | 0.1 |
| C₆ - C₈ Aromatics | 58.0 | 78.1 | 78.0 | 76.6 |
| C₉+ Aromatics | 42.1 | 16.1 | 16.6 | 16.4 |
| **Ring Retention, mol.%** **(loss)** | -- | 97.6 (2.4) | 97.9 (2.1) | 96.1 (3.9) |

The data reported in Table 4 demonstrate the advantages of continuously cofeeding sulfur. Comparing the C₆ to C₈ aromatics of Example 11 with Examples 9 and 10, it is apparent that continuously cofeeding sulfur maintained a product of higher C₆ to C₈ aromatics content. Additionally, in Examples 9 and 10 fewer C₅- and C₆+ nonaromatics (e.g. methylcyclopentane) formed and fewer aromatics were lost. Furthermore, although not reported in Table 4, the hydrogen consumption was significantly reduced with sulfur cofeed. At 134 hours on stream, with sulfur cofeed, the hydrogen consumption was 222.6 SCF/B (Example 9), at 158 hours on stream, with sulfur cofeed, the hydrogen consumption was 202.1 SCF/B (Example 10). In Example 11, after the sulfur cofeed was discontinued, the hydrogen consumption was 312.2 SCF/B.

### Example 12

In this Example the Pt/ZSM-12 catalyst A (Example 1) was compared with a Mo/zeolite beta catalyst in the conversion of a feed comprising 55 wt% toluene and 45 wt% C⁹-C₁₀ aromatics.

The beta catalyst was prepared by muller impregnation of a 65 wt% zeolite beta/35 wt% alumina extrudate with phosphoric acid and ammonium heptamolybdate to give a catalyst containing 4.2 wt% Mo and 1.7 wt% P. The activity of the catalyst was reduced by steaming for 10hrs in 100% steam at 1025°F (550°C) to give an alpha value of 17.

Prior to testing, each catalyst was pre-sulfided with a flow of 50-60cc/min of 2%H₂S/H₂ at 100 kPa at 300-400°C for 40-60 minutes.

Results obtained in processing the above feed, after about 1 week on stream, at 800°F (430°C), 300 psig (2170 kPa), a hydrogen to hydrocarbon mole ratio of 2:1, and 2.5 WHSV are shown in Table 5 below:

**Table 5**

| | Pt/ZSM-12 | Mo/beta |
|---|---|---|
| C₉ aromatic conversion | 64.8 wt% | 62.4 wt% |
| C₁₀ aromatic conversion | 44.2 wt% | 26.4 wt% |
| Xylene yield | 31.6 wt% | 29.7 wt% |
| Benzene yield | 10.6 wt% | 6.5 wt% |
| C₆-C₁₀ ring loss | 3.9 mol% | 1.3 mol% |

The Mo/beta catalyst exhibited a lowr ring loss, but converted less C₉-C₁₀ aromatics, than the Pt/ZSM-12 catalyst.

### Example 13

In this Example the Pt/ZSM-12 catalyst B (Example 2) was compared with a Cu-modified Pt/zeolite beta catalyst in the conversion of a feed comprising 47 wt% toluene and 53 wt% C⁹-C₁₀ aromatic blend.

The beta catalyst was prepared by impregnation using the incipient wetness technique of a 65 wt% zeolite beta/35 wt% alumina extrudate with a platinum and copper salt solution to give a nominal loading of 0.1 wt% Pt and 0.033 wt% Cu on the catalyst. Comparison of the benzene hydrogenation activity of the copper-containing catalyst with an identical catalyst without copper indicated that the benzene hydrogenation activity had been reduced by the copper modification. The BHA of the copper-containing catalyst was 109 moles benzene/moles Pt/hour.

Both catalysts were presulfided for 30 minutes at 430°C with a feed doped with dibutylsulfide. The results obtained in processing the above feed at 800°F (430°C), 300 psig (2170 kPa), a hydrogen to hydrocarbon mole ratio of 2:1, and 2.5 WHSV are shown in Table 6 below:

**Table 6**

| | Pt/ZSM-12 | Cu/Pt/beta |
|---|---|---|
| C₉ aromatic conversion | 54.7 wt% | 52.9 wt% |
| C₁₀ aromatic conversion | 53.3 wt% | 43.7 wt% |
| Xylene yield | 36.1 wt% | 35.3 wt% |
| Benzene yield | 6.3 wt% | 4.1 wt% |
| C₆-C₁₀ ring loss | 2.7 mol% | 5.6 mol% |

## Claims

1. A process for converting C₉+ aromatic hydrocarbons to a product which includes xylenes comprising the step of contacting a feed comprising the C₉+ aromatic hydrocarbons, together with benzene and/or toluene, with a catalyst composition comprising a zeolite selected from beta, ZSM-12 and MCM-22 and a hydrogenation component to produce a product comprising xylenes, wherein the catalyst composition having the hydrogenation component is treated with a source of sulfur or with steam to reduce its aromatic hydrogenation activity.

2. A process as claimed in claim 1 in which the steaming step is effected under conditions including an atmosphere of 5 to 100% steam and a temperature of 500 to 1200°F (260 to 650°C).

3. A process as claimed in any preceding claim in which the catalyst composition is treated with a source of a Group IB element.

4. A process as claimed in claim 3 in which the Group IB element is copper.

5. A process as claimed in any preceding claim in which the contacting step is conducted under a hydrogen partial pressure of 100 to 3000 kPa.

6. A process as claimed in any preceding claim in in which the hydrogenation component is a metal selected from Group VIII of the Periodic Table of the Elements.

7. A process as claimed in claim 6 in which the hydrogenation component is a noble metal.

8. A process as claimed in claim 8 in which the noble metal is platinum.

9. A process as claimed in any preceding claim in which the zeolite is ZSM-12 and the noble metal is platinum.

10. A process as claimed in any of claims 1 to 5 in which the zeolite is zeolite beta and the hydrogenation component is molybdenum.

11. A process as claimed in any preceding claim in which the benzene and/or toluene represents 40 % to 90 % by volume of the total feedstock.

12. A process according to any preceding claim wherein the catalyst composition is treated with the source of sulfur before said contacting step.

## Patentansprüche

1. Ein Verfahren zur Umwandlung von C₉+ aromatischen Kohlenwasserstoffen in ein Produkt welches Xylene enthält, das umfaßt den Schritt des Zusammenführens eines Einsatzstoffes, umfassend die C₉+ aromatischen Kohlenwasserstoffe zusammen mit Benzen und/oder Toluen, mit einer Katalysatormischung, welche ein Zeolith, ausgewählt aus Beta, ZSM-12 und MCM-22, und einen Hydrierungsbestandteil umfaßt, um ein Produkt, das Xylene umfaßt, herzustellen, wobei die Katalysatormischung, die den Hydrierungsbestandteil enthält, mit einer Schwefelquelle oder Dampf behandelt wird, um ihre aromatische Hydrierungswirksamkeit zu veringern.

2. Ein Verfahren gemäß Anspruch 1, in welchem der Schritt der Dampfbehandlung unter Bedingungen ausgeführt wird, die eine Atmosphäre von 5 bis 100 % Dampf und eine Temperatur von 500 bis 1200 °F (260 bis 650 °C) mit einschließen.

3. Ein Verfahren gemäß jedem vorgehendem Anspruch, in welchem die Katalysatormischung mit einer Quelle von einem IB-Gruppenelement behandelt wird.

4. Ein Verfahren gemäß Anspruch 3, in welchem das IB-Gruppenelement Kupfer ist.

5. Ein Verfahren gemäß jedem vorgehendem Anspruch, in welchem der Schritt des Zusammenführens unter einem Wasserstoffpartialdruck von 100 bis 3000 kPa durchgeführt wird.

6. Ein Verfahren gemäß jedem vorgehendem Anspruch, in welchem der Hydrierungsbestandteil ein Metall ist, das aus der Gruppe VIII des Periodensystems der Elemente ausgewählt wurde.

7. Ein Verfahren gemäß Anspruch 6, in welchem der Hydrierungsbestandteil ein Edelmetall ist.

8. Ein Verfahren gemäß Anspruch 8, in welchem das Edelmetall Platin ist.

9. Ein Verfahren gemäß jedem vorhergehenden Anspruch, in welchem der Zeolith ZSM-12 ist und das Edelmetall Platin ist.

10. Ein Verfahren gemäß jedem vorhergehenden Anspruch der Ansprüche 1 bis 5, in welchem der Zeolith der Zeolith Beta ist und der Hydrierungsanteil Molybdän ist.

11. Ein Verfahren gemäß jedem vorhergehenden Anspruch, in welchem das Benzen und/oder Toluen 40% bis 90% des Volumens des gesamten Einsatzstoffes ausmachen.

12. Ein Verfahren gemäß jedem vorhergehenden Anspruch, in welchem die Katalysatormischung vor dem besagten Zusammenführungsschritt mit einer Schwefelquelle behandelt wird.

## Revendications

1. Procédé pour convertir des hydrocarbures aromatiques en C₉+ en un produit contenant des xylènes, comprenant l'étape consistant à mettre en contact une charge d'alimentation comprenant les hydrocarbures aromatiques en C₉+, en même temps que du benzène et/ou du toluène, avec une composition de catalyseur comprenant une zéolite choisie parmi la bêta, la ZSM-12 et la MCM-22 et un constituant d'hydrogénation, pour produire un produit contenant des xylènes, dans lequel la composition de catalyseur contenant le constituant d'hydrogénation est traitée avec une source de soufre ou avec de la vapeur d'eau pour réduire son activité d'hydrogénation aromatique.

2. Procédé selon la revendication 1, dans lequel l'étape de traitement à la vapeur d'eau est effectuée dans des conditions incluant une atmosphère de 5 à 100% de vapeur d'eau à une température de 500 à 1 200°F (260 à 650°C).

3. Procédé selon l'une quelconque des revendications précédentes, dans lequel la composition de catalyseur est traitée avec une source d'un élément du groupe IB.

4. Procédé selon la revendication 3, dans lequel l'élément du groupe IB est le cuivre.

5. Procédé selon l'une quelconque des revendications précédentes, dans lequel l'étape de mise en contact est réalisée sous une pression partielle d'hydrogène de 100 à 3 000 kPa.

6. Procédé selon l'une quelconque des revendications précédentes, dans lequel le constituant d'hydrogénation est un métal choisi dans le groupe VIII du tableau périodique des éléments.

7. Procédé selon la revendication 6, dans lequel le constituant d'hydrogénation est un métal noble.

8. Procédé selon la revendication 7, dans lequel le métal noble est le platine.

9. Procédé selon l'une quelconque des revendications précédentes, dans lequel la zéolite est la ZSM-12 et le métal noble est le platine.

10. Procédé selon l'une quelconque des revendications 1-5, dans lequel la zéolite est la zéolite bêta et le constituant d'hydrogénation est le molybdène.

11. Procédé selon l'une quelconque des revendications précédentes, dans lequel le benzène et/ou le toluène représentent 40% à 90% en volume de la charge d'alimentation totale.

12. Procédé selon l'une quelconque des revendications précédentes, dans lequel la composition de catalyseur est traitée avec la source de soufre avant ladite étape de mise en contact.
